# EUROPEAN PATENT APPLICATION

(11) **EP 2 704 043 A1**
(43) Date of publication of application: **05.03.2014**
(21) Application number: 13178542.0
(22) Date of filing: 30.07.2013
(51) Int. Cl.: G06F 19/00

(54) **Medical support device and system for cooperation with medical specialists**

(30) Priority: 30.08.2012 JP 2012189955
(71) Applicant: Fujifilm Corporation, Minato-ku Tokyo 106-8620 (JP)
(72) Inventor: Ohta, Yasunori, Kanagawa (JP)
(74) Representative: Klunker . Schmitt-Nilson . Hirsch

(57) **Abstract**

A medical support device (25) distributes medical information of a patient to external terminals (11) the supporters have, and shares comments from the supporters to support diagnosis and treatment of a doctor in charge of the patient. In the medical support device (25), an information sending section (39a) distributes the information to the external terminals (11). An information receiving section (39b) receives response information sent from the doctor or the supporter in response to the distributed information. A relation display controller (36b) displays the distributed and response information in a related manner. An importance determining section (37) determines the degree of importance of the information to be distributed. When the degree of importance of the information equals or exceeds a threshold value, a reception state setting section (38) suspends transmission of new information from the external terminals (11) and puts the terminals (11) in a ready-to-receive state.

## Description

### BACKGROUND OF THE INVENTION

### 1. Field of the Invention

The present invention relates to a medical support device and system.

### 2. Description Related to the Prior Art

In recent years, medical technologies against injury and disease such as infection have been considerably advanced. The medical technologies have been specialized with its advancement, and hence generally require expertise of many medical specialists, technicians, and the like. On the other hand, a patient's condition changes with time, and there is often a case where an initial treatment has a great influence on an outcome. For the purpose of administering an optimal treatment at optimal time, a technology is demanded in which an array of medical specialists and the like are allowed to be involved in medical practice of a patient in an appropriate manner.

US Patent Application Publication No. 2009/0222286 (corresponding to Japanese Translation of PCT International Application Publication No. 2009-518732) describes a medical information storage system that stores medical history information in a database of a hospital when a patient receives a specific treatment. According to the medical history information read out from the database, medical treatments and events are arranged along a time line so that a medical history can be seen at first sight. Furthermore, detailed information is associated with each treatment or event to inform a user of a date and type of the treatment, a doctor in charge of the treatment, findings of the doctor, and the like.

US Patent Application Publication No. 2004/0230458 (corresponding to Japanese Patent Laid-Open Publication No. 2004-280807) describes a cyber-hospital system that is connected to external terminals through a network. This system allows a doctor in a remote location to make a diagnosis of a patient. This system includes a memory means, an input means, a processing means, and a sending means. The memory means stores doctors' information. The input means captures patient condition information from the external terminal. The processing means retrieves and collects information about a doctor who is described in the patient condition information from the doctors' information. The sending means sends the retrieved information of the doctor to the external terminal. When the patient condition information is inputted from the external terminal, the system designates the doctor in charge of the patient. The designated doctor makes a diagnosis based on medical information (for example, results of a blood test, a medical image, and the like) of the patient obtained from the system through the external terminal. The doctor also creates a treatment plan, and sends the treatment plan to the system. The patient can make requests to change the doctor in charge and to choose medical specialists. The doctor can make requests for cooperation of the medical specialists and to use medical examination facilities. Based on the requests, the cyber-hospital including the doctor in charge, the medical specialists, a radiologist, and the medical examination facilities is established for diagnosis and treatment by a medical team.

According to the US Patent Application Publication No. 2009/0222286, the time-series medical history information is created within a specific medical facility. The system neither intends to obtain and share the medical history information in a plurality of medical facilities and doctors through a network, nor indicates the degree of importance of each event. According to the US Patent Application Publication No. 2004/0230458, since data transmission and reception are performed between the system and each external terminal during the diagnosis by the medical team, communication information to be sent to the system and medical information to be sent from the system to the external terminals collide in the network. The collision interrupts data communication, so lack of the important medical information interferes with or delays the correct diagnosis.

### SUMMARY OF THE INVENTION

An object of the present invention is to provide a medical support system that prevents collision of information in a network and prioritizes the distribution of medical information having a high degree of importance to terminals, in order to actualize a correct and quick diagnosis.

To achieve the above and other objects, a medical support device according to the present invention includes a distribution server that distributes a participation request in a medical support operation to a supporter in response to a medical support request command sent by a doctor from a hospital terminal, and distributes the medical information including medical chart information and a test image to the doctor and the supporter. The distribution server includes an importance determining section for determining a degree of importance of the medical information, and a reception state setting section. The degree of importance is set at a predetermined initial value and changeable using the hospital terminal or the external terminal. The reception state setting section temporarily puts the hospital terminal and the external terminal in a transmission disabled state and enables only reception of the medical information, when the degree of importance of the medical information to be sent equals or exceeds a threshold value.

The medical support device further includes a management server and a terminal control server. The management server registers and deregisters the supporter, and approves participation of the supporter in the medical support operation. The terminal control server controls operation of the hospital terminal and the external terminal through a medical support application installed in the hospital terminal and the external terminal.

The distribution server preferably has a reception display command section for commanding the hospital terminal or the external terminal to automatically display a message indicating completion of reception of the medical information on a monitor of the hospital terminal or the external terminal. The distribution server preferably has an information display command section for commanding the hospital terminal or the external terminal to automatically display the medical information received from the distribution server on the monitor of the hospital terminal or the external terminal.

The reception display command section preferably commands at least one of: to blink the message indicating completion of reception, to change color of the message indicating completion of reception, to change a typeface of the message indicating completion of reception, and to change a font size of the message indicating completion of reception. The information display command section preferably commands at least one of: to blink display of the received medical information, to change color of the display of the received medical information, to change a typeface of the display of the received medical information, and to change a font size of the display of the received medical information. A display format of the hospital terminal or the external terminal is preferably customized by its user.

Condition description or medical education may be carried out between the doctor and the supporter using the medical information having the degree of importance that equals or exceeds the threshold value.

A medical support system according to the present invention includes a hospital terminal operated by a doctor in charge of treatment, an electronic medical chart server for providing medical chart information, a test image server for providing a test image, an external terminal that a supporter has, and the medical support device described above.

According to the present invention, when the distribution server distributes the medical information having a high degree of importance, the hospital terminal and the external terminal are temporarily put in the transmission disabled state and allowed only to receive the medical information. Therefore, it is possible to reliably distribute the important medical information to the hospital terminal and the external terminal. Sharing the important information between the doctor and the supporter facilitates making a correct diagnosis and producing an appropriate treatment plan.

### BRIEF DESCRIPTION OF THE DRAWINGS

For more complete understanding of the present invention, and the advantage thereof, reference is now made to the subsequent descriptions taken in conjunction with the accompanying drawings, in which:
Fig. 1 is a schematic view of a medical support system;
Fig. 2 is a functional block diagram of a distribution server of a medical support device;
Fig. 3 is a block diagram of an external terminal;
Fig. 4 is a medical support request issuing screen displayed on a monitor of a hospital terminal;
Fig. 5 is a medical support request receiving screen displayed on a monitor of the external terminal;
Fig. 6 is a relation display screen displayed on the monitor of the external terminal;
Fig. 7 is a comment screen displayed on the monitor of the external terminal;
Fig. 8 is a test image screen displayed on the monitor of the external terminal;
Fig. 9 is a general screen displayed on the monitor of the hospital terminal;
Fig. 10 is a flowchart of the medical support system showing a procedure from an issue of a medical support request to a decision of participation in medical support;
Fig. 11 is a flowchart of the medical support system showing a procedure from transmission and distribution of medical information to completion of the medical support;
Fig. 12 is a flowchart of an information distribution process performed by the distribution server; and
Fig. 13 is a flowchart of a display style change process performed by the distribution server.

### DESCRIPTION OF THE PREFERRED EMBODIMENTS

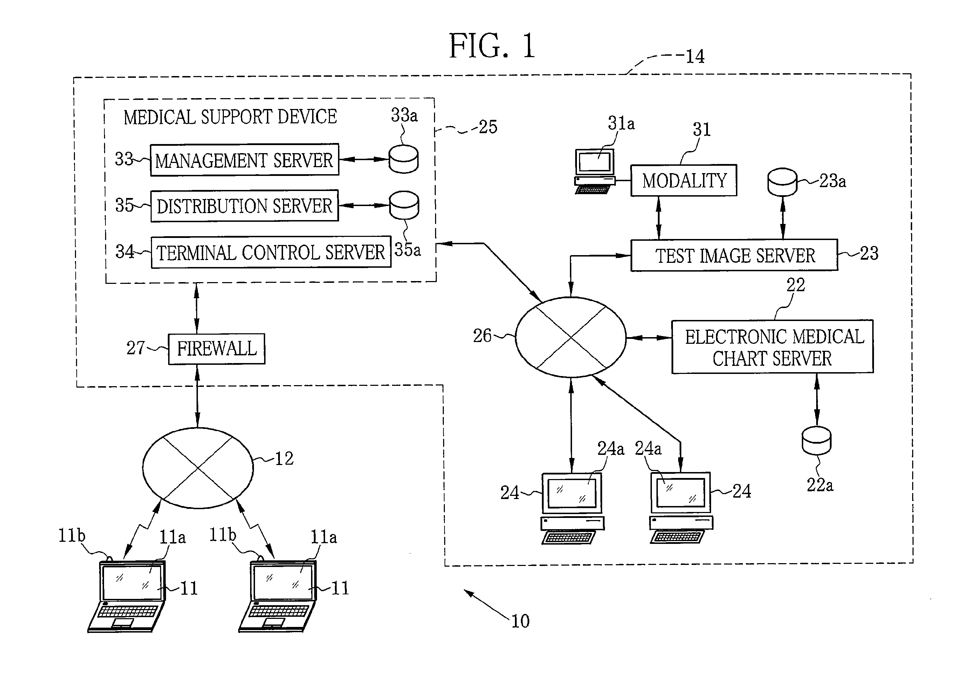

In Fig. 1, a medical support system 10 being an embodiment of the present invention supports medical diagnosis and treatment of a doctor who is in charge of a patient (protomedicus, hereinafter simply called "doctor") by using external terminals 11 that registered supporters (hereinafter simply called "supporters") have. This medical support system 10 starts a medical support operation according to individual patients, including outpatients and emergency patients, in response to a medical support request made by the doctor. In the medical support operation, the doctor and medical specialists being the supporters can exchange opinions based on medical information. The medical information sent by the doctor, the supporter, or the like is attached with a degree of importance determined by a sender. According to the degree of importance, a distribution method of the medical information is changed by which the medical information is distributed from a distribution server 35 to the doctor and supporters. The degree of importance is, for example, an index on a scale of 1 to 10 attached to the medical information as a numerical value. An index of "1" indicates the lowest degree of importance, and an index of "10" indicates the highest degree of importance.

The medical support system 10 is constituted of a hospital system 14 established within a hospital and the external terminals 11 connected to the hospital system 14 through a network 12. The supporters include not only the medical specialists excluding the doctor, such as physicians, surgeons, nurses, and laboratory technicians, but also a hospital clerical staff.

Each supporter has the external terminal 11. The external terminal 11 is, for example, a notebook PC that is installed with an application specific to the medical support system (hereinafter called "medical support application") and connected to the Internet through a wireless LAN. A medical support request receiving screen and various screens for use in the medical support operation are displayed on a monitor 11a of the external terminal 11. Various commands and text are entered using a mouse, a keyboard, or the like of the external terminal 11 in the medical support operation.

The external terminal 11 is provided with a red LED 11b. The external terminal 11 may be further provided with a speaker or a siren (neither shown). While the supporter participates in the medical support operation, the red LED 11b lights up or blinks in order to inform people around the supporter of his/her participation. The speaker may announce that "participating in the medical support operation" or the like. The siren may beep to announce the participation.

An arbitrary device e.g. a personal digital assistant (PDA) or a portable terminal is usable as the external terminal 11, as long as it has communication, display, and input functions. The external terminal 11 may be a desktop PC if portability is unnecessary, or a smartphone (intelligent portable telephone) having a touch screen if portability is necessary.

The external terminals 11 are connected to the hospital system 14 through the network 12. Any type of network, e.g. the Internet, an exclusive line, a portable telephone line, a public wireless LAN, or a mixed network of above is usable as the network 12 as long as it communicatably connects the external terminals 11 and the hospital system 14. The external terminals 11 can communicate with the hospital system 14 through the network 12 in remote locations outside or inside the hospital. The external terminals 11 are connected to the hospital system 14 through a VPN (virtual private network), for example, to prevent information leakage.

The hospital system 14 is constituted of an electronic medical chart server 22, a test image server 23, hospital terminals 24, a medical support device 25, a hospital LAN 26 for connecting the servers 22 and 23, the terminals 24, and the device 25 described above, and a firewall 27 interposed between the medical support device 25 and the network 12. In addition to the electronic medical chart server 22 and the test image server 23, for example, a server for providing a result of a blood test or a pathological test may be used as an information providing server.

The hospital LAN 26 is a network established in the hospital. The firewall 27 permits only communications for the medical support operation between the external terminals 11 and the medical support device 25 through the network 12. The firewall 27 blocks an unauthorized access to the medical support device 25 and to the server 22, 23 or the terminal 24 connected to the hospital LAN 26, in order to prevent leakage and tampering of information.

The hospital terminals 24 are located in consultation rooms and treatment rooms of individual departments, emergency rooms, and the like. The doctor operates the hospital terminal 24. The hospital terminal 24 is a personal computer that is installed with applications composed of an electronic medical chart program, a test request program, and a medical support operation program, and the like. The doctor makes with the hospital terminal 24 a reference and an input to an electronic medical chart, a radiography test request, a medical support request, a comment in the medical support operation, and the like. The hospital terminal 24 displays on its monitor 24a the electronic medical chart, test images, a medical support request issuing screen, and various screens for participation in the medical support operation. The hospital terminal 24 accesses the medial support device 25 to register or deregister the supporter.

The electronic medical chart server 22 stores basic information and medical chart information of each patient on its database 22a, and provides the information as necessary. The basic information includes a patient ID, a name, a birthday, a sex, a height, a weight, and the like of the patient. In the case of a new patient, a new patient ID is automatically produced by entering the basic information of the patient on a registration terminal (not shown).

The electronic medical chart server 22 writes the medical chart information to the database 22a. The medical chart information includes findings of the doctor, medication data, and the like that are inputted from the hospital terminal 24 to the electronic medical chart. The medical chart information includes the patient ID and a reception ID issued by a reception terminal (not shown) upon checking in. The medical chart information includes a doctor ID of the doctor in charge of the patient and a test image and its image ID if the test image exists.

The test image server 23 is connected to modality 31 such as a CT system and an MR system. The test image server 23 stores test images such as CT images obtained by the modality 31 on its database 23a, and provides the test images as necessary. The test image server 23 receives the test request from the hospital terminal 24, and displays instructions for the test on an operation terminal 31a of the modality 31. When a radiological technician completes imaging the patient with the modality 31, an obtained test image is written to the database 23a together with its image ID, an imaging date and time, a technician ID of the radiological technician, the reception ID, the patient ID, and the like.

Upon starting the medical support operation, the medical support device 25 informs the electronic medical chart server 22 and the test image server 23 of the reception ID and the patient ID. Thus, whenever the electronic medical chart corresponding to the reception ID is updated, the electronic medical chart server 22 sends the updated contents of the electronic medical chart to the medical support device 25. Whenever a new test image corresponding to the patient ID is obtained, the test image server 23 sends the test image to the medical support device 25 with attaching information related to a degree of importance of the test image. The medical support device 25 automatically distributes the updated contents of the electronic medical chart or the new test image to the external terminals 11 and the hospital terminal 24, whenever receiving it. Note that, the new information may be chosen and distributed by manual operation of the doctor.

The medical support device 25 is constituted of a management server 33, a terminal control server 34, the distribution server 35, and the like. The management server 33 performs the registration and deregistration of the supporter and approval (confirmation and record) for participation of the supporter in the medical support operation. The management server 33 has a database 33a on which supporter information is stored. The hospital terminal 24 or a management terminal (not shown) accesses the management server 33 to register or deregister the supporter and modify the supporter information. To register a new supporter, the management server 33 writes new supporter information to the database 33a. To deregister a supporter, the management server 33 deletes his/her information from the database 33a.

The supporter information includes a supporter ID, a name, a medical license e.g. physician, surgeon, nurse, or radiological technician, a specialization, and an authentication ID and a password of the supporter, the type and an unique ID number of the external terminal 11 used by the supporter, an address of the external terminal 11, and the like. As the unique ID number, for example, a MAC address provided to a network interface of the external terminal 11, a number called "IMSI" recorded to a SIM card of a portable telephone, or the like is usable. The address of the external terminal 11 is an IP address, an e-mail address, a phone number of the portable telephone, or the like.

Note that, a plurality of external terminals 11 and their addresses may be recorded for the single supporter, so that the supporter can use the plurality of external terminals 11. A plurality of unique ID numbers such as the MAC address and IMSI may be recorded for the single external terminal 11. The approval for the external terminal 11 may be performed using any one of the unique ID numbers according to a communication method adopted by the external terminal 11.

When the supporter participates in the medical support operation, the supporter inputs his/her authentication ID and password to the external terminal 11. The inputted authentication ID and password are sent to the management server 33. The unique ID number of the used external terminal 11 is automatically sent to the management server 33. The management server 33 judges whether or not the authentication ID, the password, and the unique ID number coincide with those registered in advance. When the authentication ID, the password, and the unique ID number coincide, the management server 33 approves an operator of the external terminal 11 as a participant in the medical support operation.

When a medical support request command is issued from the hospital terminal 24, the management server 33 accesses the database 33a and retrieves the addresses of the supporters who the doctor has designated as destinations. The retrieved addresses are sent to the distribution server 35.

The terminal control server 34 controls the external terminals 11 through the medical support application. For example, the terminal control server 34 deletes distributed information stored in the external terminals 11 after the completion of a single medical support operation.

The distribution server 35 distributes the medical information attached with information (importance information) related to a degree of importance of the medical information itself to the external terminals 11. The medical information includes the medical support request, a comment made in the medical support operation, the updated contents of the electronic medical chart, and the like. In response to the medical support request command inputted by the doctor from the hospital terminal 24, the distribution server 35 distributes the medical support request to the external terminals 11 of the supporters who the doctor has designated. The medical support request includes initial information (request time information) described later on.

The initial information includes the patient ID, the reception ID, reception time, a medical support request comment inputted by the doctor, the test images obtained by the modality 31, the contents of the electronic medical chart, and the like. The distribution server 35 retrieves the contents of the electronic medical chart from the electronic medical chart server 22 by using, for example, the reception ID as a key. The distribution server 35 also retrieves the test images from the test image server 23 by using the image IDs as keys. Each of the medical support request comment, the test images, and the contents of the electronic medical chart is automatically attached with the importance information at the time of creation. The degree of importance is set at "7" as an initial value on a scale of 1 to 10, and is changeable separately by the doctor.

After the start of the medical support operation, the distribution server 35 receives a comment by the doctor from the hospital terminal 24, a comment by the participant from the external terminal 11, the updated contents of the electronic medical chart by the doctor, and the new test image from the test image server 23 together with the importance information attached thereto. The distribution server 35 distributes the comments, the updated contents of the electronic medical chart, and the new test image to the external terminals 11 of the participants and the hospital terminal 24 together with additional information including the importance information. The distribution server 35 has a participant list that contains the addresses of the external terminals 11 to which the information is to be distributed. The address of the external terminal 11 of the approved supporter is added to the participant list. The address of the supporter who has sent a notice of non-participation is deleted from the participant list.

The additional information of the comment includes a date and time of making the comment and a name of a commenter. The name of the commenter may be identified as a name of the supporter who has the external terminal 11 that sent the comment. The additional information of the test image includes the image ID, a date and time of imaging, the type of the modality 31, and the like. Note that, the test image is compressed into an appropriate size before the distribution.

The distribution server 35 is connected to a database 35a. The database 35a stores the initial information and the information distributed to the external terminals 11 and the hospital terminal 24 together with its importance information attached at the time of distribution. The distributed information includes the reception ID, a distribution ID, dates and time of receiving and distributing the medical information (comment and test image), the supporter ID of the commenter, and the like. In the case of the test image, the distributed information includes the image ID and a device ID of the used modality 31.

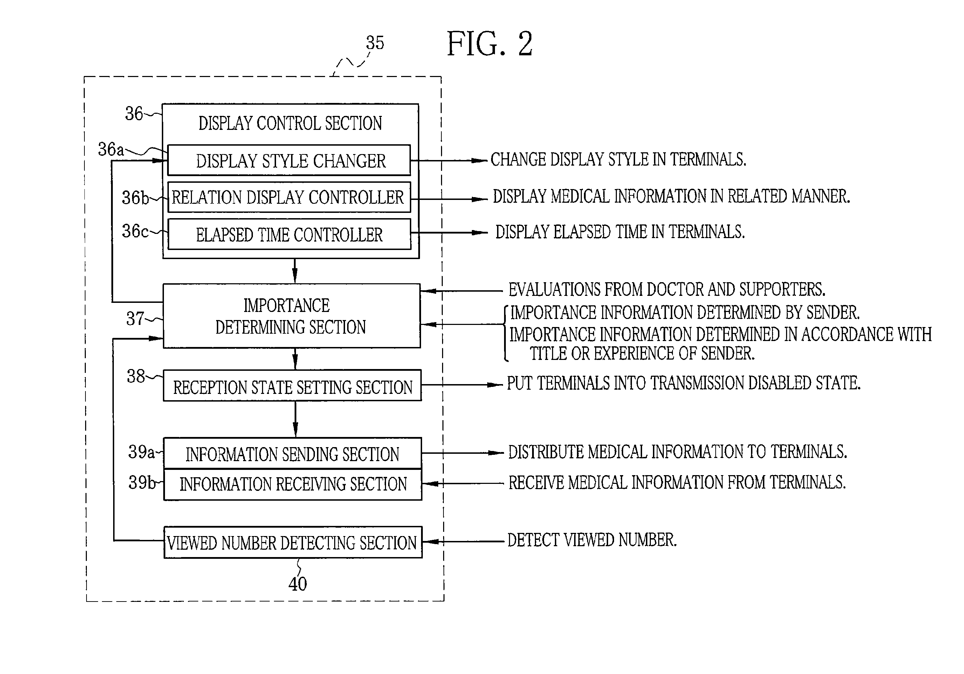

As shown in Fig. 2, the distribution server 35 is constituted of a display control section 36, an importance determining section 37, a reception state setting section 38, an information sending section 39a, an information receiving section 39b, and a viewed number detecting section 40. The display control section 36 controls display of the external terminals 11 and the hospital terminal 24. The display control section 36 is provided with a display style changer 36a, a relation display controller 36b, and an elapsed time controller 36c. The display style changer 36a commands each terminal to change a display style of the information in each terminal in accordance with the degree of importance. The relation display controller 36b commands each terminal to display the information in a related manner. The elapsed time controller 36c manages elapsed time since the occurrence of an event causing the medical support operation, i.e. the occurrence of an attack or an accident, and displays the elapsed time on each terminal. The display style changer 36a has the functions of blinking the display of the information on each terminal, changing text color in the display of the information on each terminal, changing a typeface in the display of the information on each terminal, and changing a font size in the display of the information on each terminal. The display control section 36 functions as a reception display command section, which commands the hospital terminal 24 and the external terminals 11 to automatically display a message indicating the completion of reception of the information from the information sending section 39a to each terminal 24 or 11 on the monitor 24a or 11a of each terminal 24 or 11. The display control section 36 also functions as an information display command section, which commands the hospital terminal 24 and the external terminals 11 to automatically display the information sent from the information sending section 39a to each terminal 24 or 11 on the monitor 24a or 11a of each terminal 24 or 11.

The importance determining section 37 detects the degree of importance attached to the medical information such as the comment or the test image. When the degree of importance attached to the medical information equals or exceeds a predetermined threshold value, for example, when the degree of importance is 7 or more, the importance determining section 37 judges the medical information to be important. In this case, the reception state setting section 38 puts the terminals 24 and 11 that are to receive the important medical information in a ready-to-receive state. In the ready-to-receive state, the transmission of new medical information from each terminal 24 or 11 is temporarily suspended, and the terminals 24 and 11 wait for receiving the important medical information. The information sending section 39a sends the important medical information to each terminal 24 or 11. The information sending section 39a sends the medical support request to the external terminal 11 of every supporter, and then distributes the updated medical information including information of a latest patient's condition to the supporters (participants) who consented to participate, whenever the updated medical information is obtained. The information receiving section 39b receives the medical information from the management server 33 or each terminal 24 or 11. The viewed number detecting section 40 detects whether or not a receiver of the medical information has viewed the medical information.

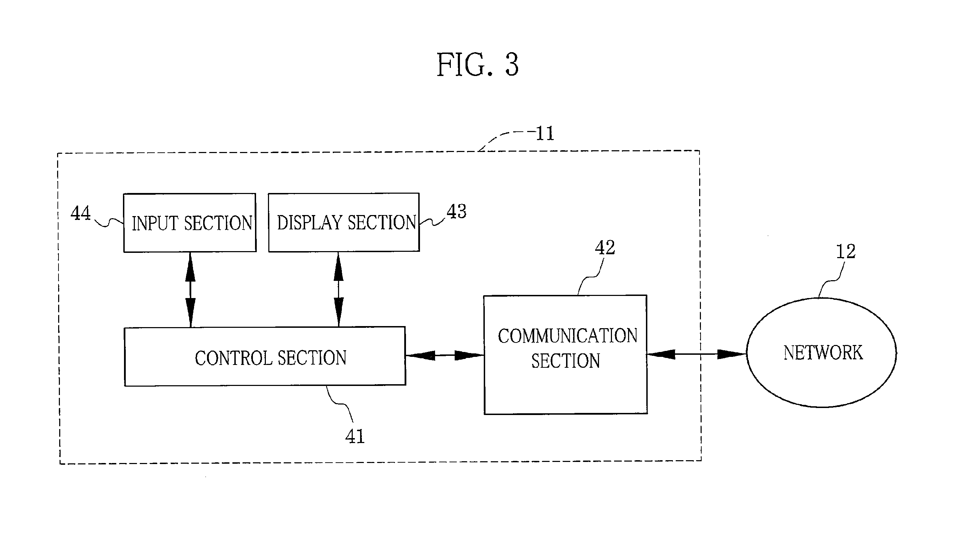

As shown in Fig. 3, the medical support application is installed in the external terminal 11, as described above. By executing the medical support application, the external terminal 11 functions as a control section 41, a communication section 42, a display section 43, and an input section 44 by executing the medical support application. The control section 41 has centralized control over the communication section 42, the display section 43, and the input section 44. The communication section 42, including a network interface and the like, establishes communication with the medical support device 25 through the network 12 and the firewall 27. The display section 43 includes the monitor 11a to display the medical support request receiving screen and the screens related to the medical support operation. The input section 44 includes the mouse, the keyboard, and the like.

Part of the control section 41 and the communication section 42 always works, so that the external terminal 11 receives the medical support request from the medical support device 25 anytime even in a sleep state. Upon receiving the medical support request by the communication section 42, the control section 41 displays the medical support request receiving screen on the display section 43. The supporters are preferably informed of the reception of the medical support request by sound, vibration, blinks of the red LED 11b, or the like.

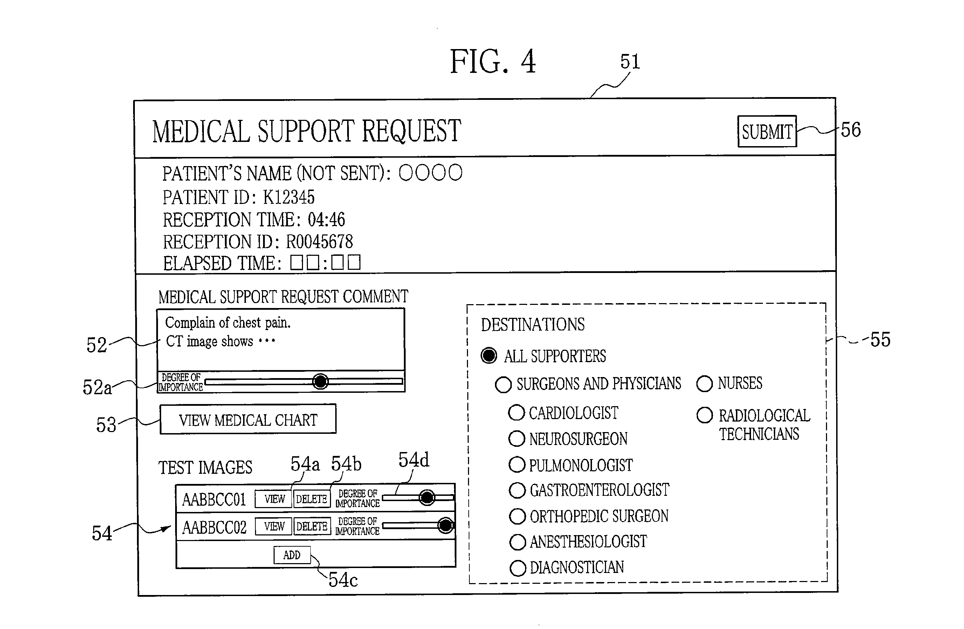

As shown in Fig. 4, a medical support request issuing screen 51 is displayed on the monitor 24a of the hospital terminal 24. The medical support request issuing screen 51 is displayed in response to performing predetermined operation in, for example, an electronic medical chart screen on the hospital terminal 24. Based on input and settings of the medical support request issuing screen 51, the medical support request command is sent from the hospital terminal 24 to the medical support device 25.

In the medical support request issuing screen 51, "patient's name", "patient ID", "reception time", "reception ID", and "elapsed time" are automatically retrieved from the database 22a of the electronic medical chart server 22. Note that, the "patient' s name" is not distributed to the external terminals 11 for privacy reason, while the other items i.e. "patient ID", "reception time", "reception ID", and "elapsed time" are distributed in the medical support request.

The doctor fills out a comment input box 52 with the medical support request comment. There is provided an importance setting field 52a just under the comment input box 52 so that the doctor himself/herself can set the degree of importance of the medical support request comment on a scale of 1 to 10. By a click on a medical chart view button 53, a window that displays the contents of the electronic medical chart distributed at the time of medical support request, which includes a symptom of the patient, findings of the doctor, and medical progress is open. Also, there is provided an importance setting field (not shown) for setting the degree of importance of the contents of the electronic medical chart on a scale of 1 to 10.

In the medical support request issuing screen 51, there is a test image list 54, which lists the image IDs of the test images distributed in the medical support operation. Upon opening the medical support request issuing screen 51, the hospital terminal 24 automatically accesses the test image server 23, and retrieves the image IDs of the test images by using the reception ID as a key. The retrieved image IDs are listed on the test image list 54.

In the test image list 54, each image ID is provided with a view button 54a and a delete button 54b. Upon a click of the view button 54a, the corresponding test image is obtained from the test image server 23 and displayed on the monitor 24a. A click of the delete button 54b deletes the image ID from the test image list 54.

The test image list 54 is provided with an add button 54c, which is used for adding another test image to the test image list 54. By a click of the add button 54c, a test image selection screen is displayed. The test image to be distributed is selected in the test image selection screen, and the image ID of the selected test image is added to the test image list 54. Thus, it is possible to distribute a past test image and the like of the same patient.

There is provided an importance setting field 54d in the test image list 54. The doctor can change the degree of importance of each test image from its initial value "7".

The medical support request issuing screen 51 includes a destination designating section 55 to designate one or more destinations of the medical support request. In this embodiment, the destination can be designated in accordance with the medical license (attribute) and the specialization, in addition to designating all supporters as the destinations. As for the medical license, the supporters are categorized as "surgeons and physicians", "nurses", and "radiological technicians". As for the specialization, the surgeons and physicians are categorized as "cardiologist", "neurosurgeon", "pulmonologist", and the like. A plurality of categories can be designated at the same time.

Upon a click of a submit button 56, the hospital terminal 24 sends the medical information and the like inputted and set in the medical support request issuing screen 51 to the medical support device 25. The destination information designated in the medical support request issuing screen 51 is sent to the management server 33. The reception ID, the medical support request comment and the importance information attached thereto, and the test images with the image IDs and the importance information attached thereto are sent to the information receiving section 39b of the distribution server 35. The information received by the information receiving section 39b is distributed to the designated destinations by the information sending section 39a.

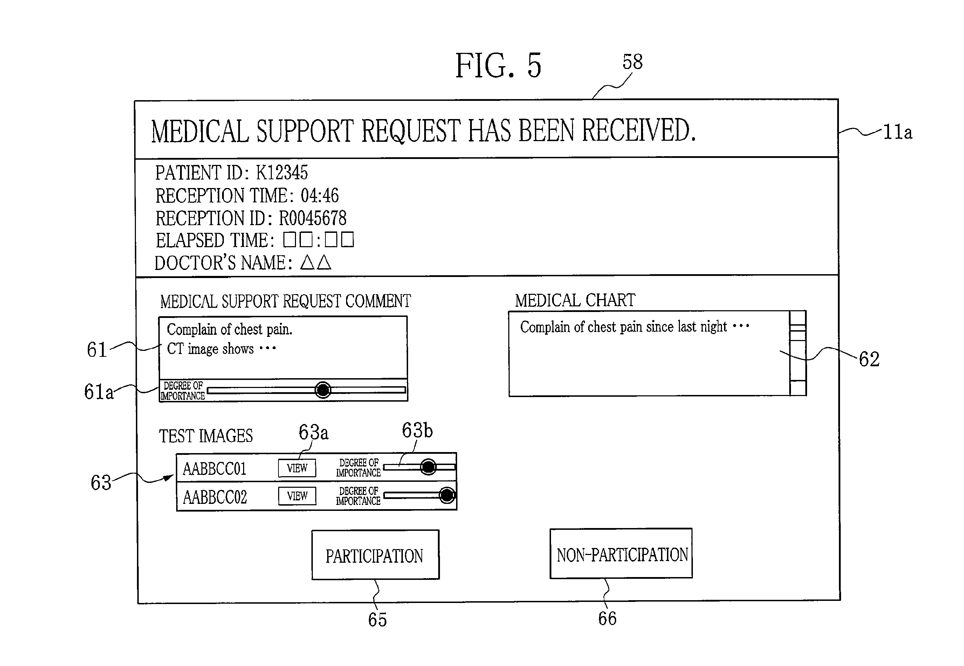

The information sending section 39a of the distribution server 35 sends the medical support request to the external terminals 11. Thus, as shown in Fig. 5, a medical support request receiving screen 58 is displayed on the monitor 11a of each external terminal 11. In the medical support request receiving screen 58, the patient ID, the reception time, the reception ID, the elapsed time, a medical support request comment 61 and the degree of importance thereof, the contents of the electronic medical chart 62, and a test image list 63 and the degree of importance of each test image are displayed as the initial information. The degree of importance of the medical support request comment 61 is shown in an importance showing field 61a. The degree of importance of each test image is shown in an importance showing field 63b. By a click of a view button 63a provided in each image ID of the test image list 63, the test image is viewed in a different window.

The medical support request receiving screen 58 displayed in the external terminal 11 is provided with a participation button 65 and an non-participation button 66. A click of the participation button 65 opens an authentication screen (not shown). The supporter inputs the authentication ID and the password to the authentication screen. When the management server 33 of the medical support device 25 approves the authentication, the supporter becomes the participant. When the supporter clicks the non-participation button 66, the medical support device 25 is notified of the non-participation of the supporter.

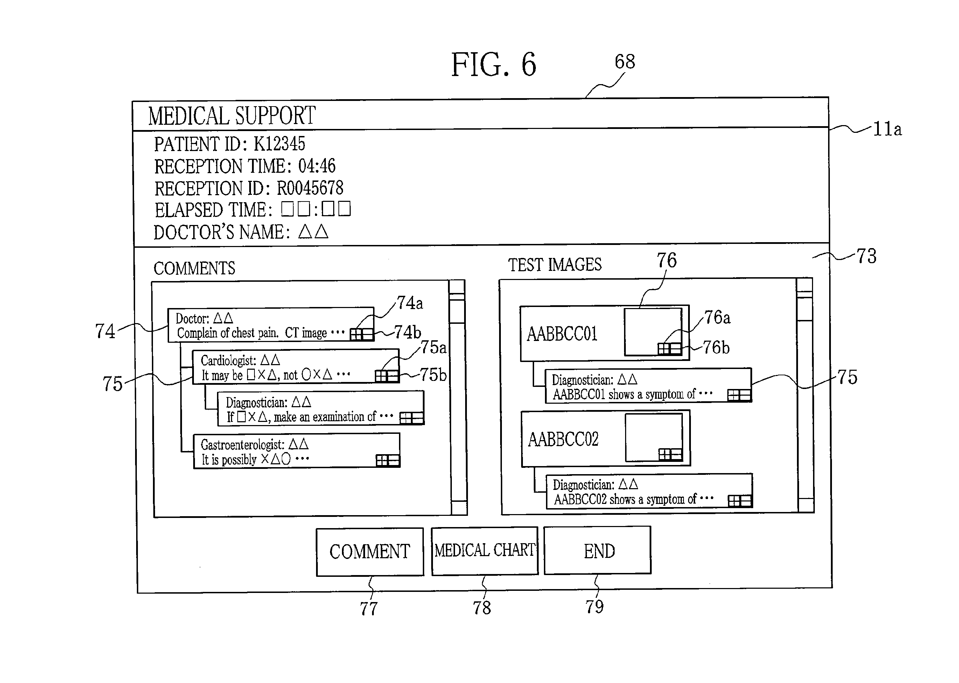

Upon starting the medical support operation, the comments from the doctor and the supporters are collected from the hospital terminal 24 and the external terminals 11 to the medical support device 25 whenever each comment is made, and a relation display screen 68 is produced as shown in Fig. 6. This relation display screen 68 is displayed on the monitor 11a of the external terminal 11. In the relation display screen 68, the comments made by the doctor and the supporters are displayed in a manner related to each other or to the test images. The relation display screen 68 has an information display section 73 that has a comment list field and a test image list field. The comment list field displays the medical support request comment, another comment made on the medical support request comment, and further another comment. The test image list field displays a test image and a comment made on the test image. In this embodiment, the comments are displayed so as to show the relation that each comment is based on. Note that, a way of displaying the medical support request comment, the other comments, and the test images is not limited to this.

A comment box 74 for displaying the medical support request comment is at the top of the comment list field. Under the comment box 74, another comment box 75 is displayed. Another comment made by the supporter on the medical support request comment is displayed in the comment box 75 in a manner related to the comment box 74. Further another comment made by the supporter on the comment displayed in the comment box 75 is displayed under the comment box 75 in a related manner. In each of the comment boxes 74 and 75, a name of a commenter, a comment date and time, and the like are displayed together with the comment itself.

The comment box 74 has evaluation buttons 74a and 74b by which each participant can evaluate the contents of the medical support request comment. The comment box 75 has evaluation buttons 75a and 75b by which each participant can evaluate the contents of the comment displayed in the comment box 75. The evaluation buttons 74a and 75a are clicked to evaluate the comment as the important information. A single click of the evaluation button 74a or 75a increases the degree of importance by a predetermined rank, for example, one level. The evaluation buttons 74b and 75b are clicked to evaluate the comment as unimportant information. A single click of the evaluation button 74b or 75b decreases the degree of importance by one level, for example. Note that, the relation display screen 68 has an area (not shown) for showing the degree of importance of each comment. The degree of importance set by the clicks of the evaluation buttons 74a, 74b, 75a, and 75b is displayed in this area. The clicks of the evaluation buttons 74a, 74b, 75a, and 75b are sent from the external terminal 11 of an evaluator to the importance determining section 37 of the distribution server 35 through the network 12. Since the importance information on the comment is sent from the external terminal 11 of every evaluator to the importance determining section 37, the importance information made by a sender of the comment and the importance information made by the evaluators are collected to the distribution server 35. Based on the collected importance information, the degree of importance of each of the medical support request comment and the other comments is determined. The display control section 36 commands each terminal 11 or 24 to change the display style of each comment based on the determined degree of importance.

In the test image list field, thumbnail images 76 of the test images are displayed in time sequence. Under each thumbnail image 76, a comment box 75 is displayed to show a comment made on the test image of the thumbnail image 76. A commenter, a date and time of making the comment, and the like are displayed in the comment box 75.

To produce the thumbnail image 76, the display section 43 downsizes the test image distributed by the information sending section 39a of the distribution server 35. A click of the thumbnail image 76 opens a test image display screen for displaying the test image of the thumbnail image 76. Note that, the display control section 36 of the distribution server 35 may produce the thumbnail image 76, and the information sending section 39a may distribute the thumbnail image together with the test image.

Each thumbnail image 76 has evaluation buttons 76a and 76b by which each participant evaluates the contents of the test image. The evaluation button 76a is clicked to evaluate the test image of the thumbnail image 76 as the important information. A single click of the evaluation button 76a increases the degree of importance by a predetermined rank, for example, one level. The evaluation button 76b is clicked to evaluate the test image as unimportant information. A single click of the evaluation button 76b decreases the degree of importance by one level, for example. Note that, the relation display screen 68 has an area (not shown) for showing the degree of importance of each test image. The degree of importance set by the clicks of the evaluation buttons 76a and 76b is displayed in this area. The clicks of the evaluation buttons 76a and 76b are sent from the external terminal 11 of an evaluator to the importance determining section 37 of the distribution server 35 through the network 12. As with the importance information on the comments, the importance information on the test images is collected to the distribution server 35. The display control section 36 commands each terminal 11 or 24 to change the display style of each test image based on the degree of importance, as in the case of the medical support request comment and the other comments.

The display section 43 updates the display of the comment list field and the test image list field, whenever the information sending section 39a of the distribution server 35 distributes the new medical information. Note that, instead of the relation display screen 68, the comment boxes 74 and 75 and the thumbnail images 76 may be displayed in time sequence. In this case, the relation of the medical information is not assured, but the chronology of the comments and the test images becomes clear.

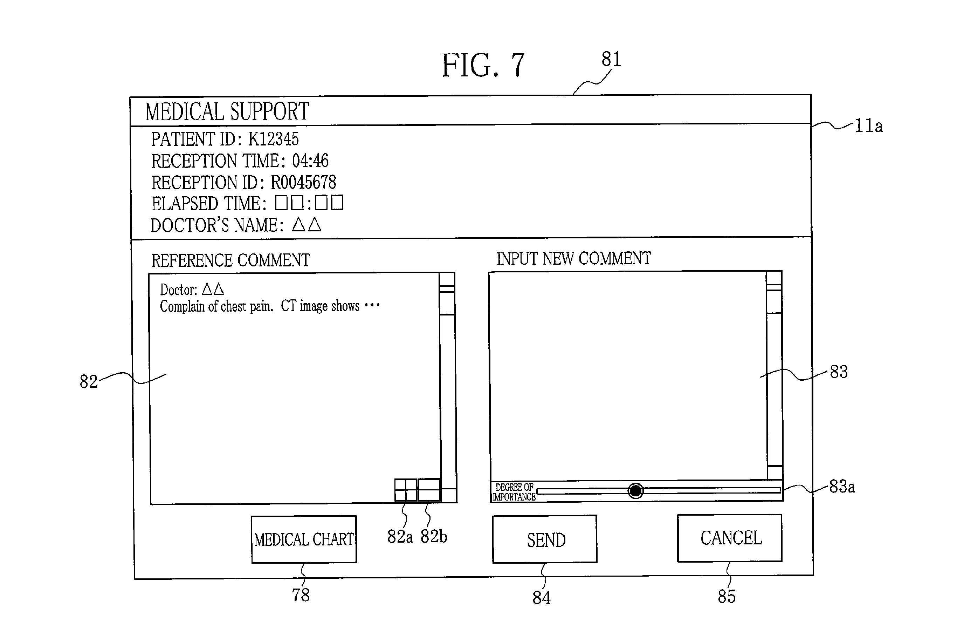

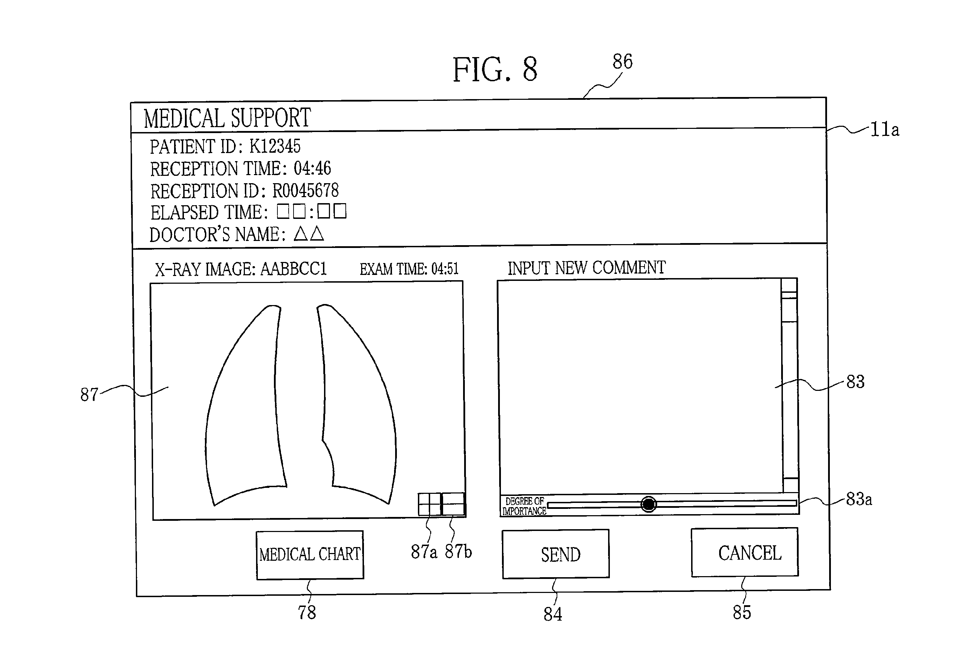

The patient ID, the reception time, the reception ID, the elapsed time, and the name of the doctor who made the medical support request are displayed above the information display section 73. There are a comment button 77, a medical chart view button 78, and an end button 79 in a lower portion of the information display section 73. By a click of the comment button 77, a comment input screen pops up. A click of the medical chart view button 78 displays the contents of the electronic medical chart. The end button 79 is clicked, when the supporter leaves the medical support operation. Upon a click of the end button 79, the control section 41 sends a leave notice to the medical support device 25. The information sending section 39a of the distribution server 35 stops the distribution of the medical information to the external terminal 11 that has issued the leave notice. Note that, the comment input screen (not shown) is a screen for inputting and sending a comment, and is constituted of a comment input field 83, a send button 84, and a cancel button 85 (all shown in Figs. 7 and 8).

When the comment box 74 or 75 is clicked in the relation display screen 68, a comment screen 81, as shown in Fig. 7, pops up in the display section 43. The comment screen 81 has a comment display field 82, which displays the comment shown in the clicked comment box 74 or 75, and the comment input field 83, in addition to the patient ID, the reception time, the reception ID, and the elapsed time.

The comment display field 82 is provided with evaluation buttons 82a and 82b by which each participant evaluates the contents of the comment. The evaluation button 82a is clicked to evaluate the comment as important information. The evaluation button 82b is clicked to evaluate the comment as unimportant information. The evaluation buttons 82a and 82b have the same function as the evaluation buttons 74a, 74b, 75a, and 75b, so the detailed description thereof will be omitted.

A new comment is inputted to the comment input field 83 using the keyboard or the like of the input section 44. An importance setting field 83a is provided just under the comment input field 83 so that a commenter himself/herself can set the degree of importance of the inputted new comment on a scale of 1 to 10.

Upon a click of the send button 84, the control section 41 sends the new comment inputted to the comment input field 83 to the medical support device 25 through the communication section 42 together with its importance information set by the commenter. The information receiving section 39b receives the contents of the comment, and the importance determining section 37 receives the importance information. Then, the information sending section 39a distributes the new comment and the importance information to the external terminals 11 of the participants and the hospital terminal 24 of the doctor. The cancel button 85 is clicked to close the comment screen 81 and go back to the screen displayed immediately before the comment screen 81. The comment screen 81 has a medical chart view button 78. By a click of the medical chart view button 78, the contents of the electronic medical chart are viewed during the input of the new comment. Note that, the comment screen 81 may be provided with a test image view button to see the test image.

When the thumbnail image 76 or the comment box 75 displayed under the thumbnail image 76 is clicked in the relation display screen 68, a test image screen 86, as shown in Fig. 8, pops up in the display section 43. The test image screen 86 displays a test image 87 corresponding to the clicked thumbnail image 76 or comment box 75 and a comment input field 83 similar to that of the comment screen 81, in addition to the patient ID, the reception time, the reception ID, and the elapsed time. In the test image 87, the type of the test image such as an X-ray image or a CT image, the image ID, and a date and time of imaging are displayed.

The participant looks at the test image 87 in detail on the test image screen 86. In the case of a CT image or an MRI image, a toolbar is displayed so that arbitrary cross sections and orientations of the test image 87 can be seen.

The test image 87 has evaluation buttons 87a and 87b by which each participant can evaluate the test image 87. The evaluation button 87a is clicked to evaluate the test image 87 as important information. The evaluation button 87b is clicked to evaluate the test image 87 as unimportant information. The evaluation buttons 87a and 87b have the same functions as the evaluation buttons 76a and 76b, so the detailed description thereof will be omitted.

The test image display screen 86 has the send button 84, the cancel button 85, and the medical chart view button 78. These buttons 84, 85, and 78 have the same functions as the buttons 84, 85, and 78 of the comment screen 81. Note that, the test image screen that is displayed by a click of the view button 63a of the medical support request receiving screen 58 does not have the comment input field 83, the send button 84, and the cancel button 85.

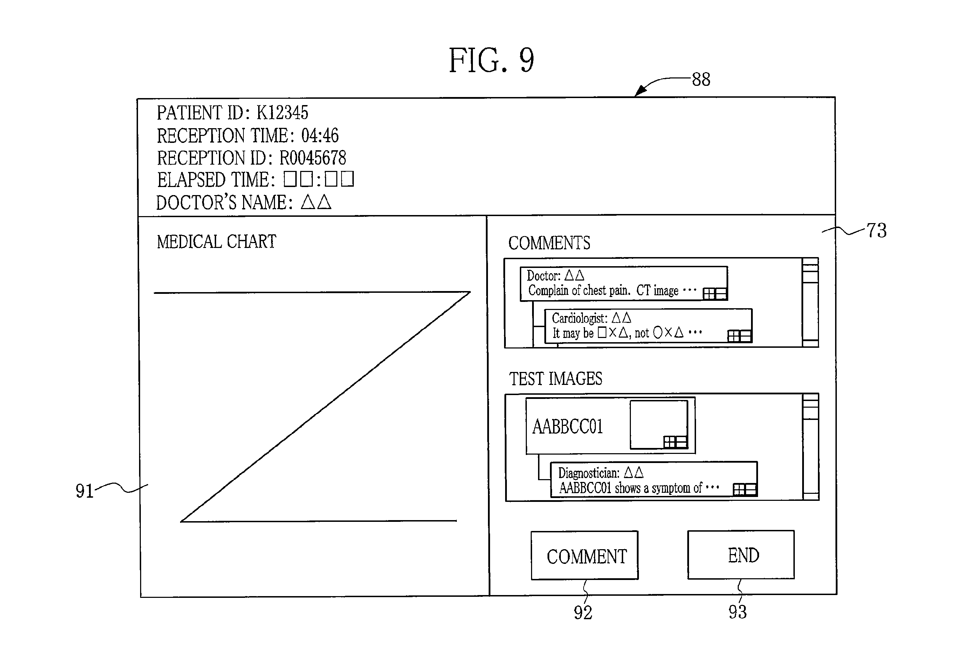

After the start of the medical support operation, as shown in Fig. 9, a general screen 88 including an electronic medical chart 91 and the information display section 73 is displayed on the monitor 24a of the hospital terminal 24. Since the same information display section 73 is displayed on both the external terminal 11 and the hospital terminal 24, the doctor can easily understand the contents of the comments from the participants and the like. With the use of the evaluation buttons 74a, 74b, 75a, and 75b of the comment boxes 74 and 75, it is possible to evaluate the contents of the comments from the hospital terminal 24. The evaluation buttons 74a, 74b, 75a, and 75b are the same as those displayed on the monitors 11a of the external terminals 11, so the detailed description thereof will be omitted.

The general screen 88 has a comment button 92 and an end button 93. By a click of the comment button 92, the doctor can input and issue a comment. The issued comment is sent to the medical support device 25 through the hospital LAN 26. The end button 93 sends an end notice of the medical support operation from the hospital terminal 24 to the medical support device 25. In response to the end notice, the medical support device 25 completes the medical support operation.

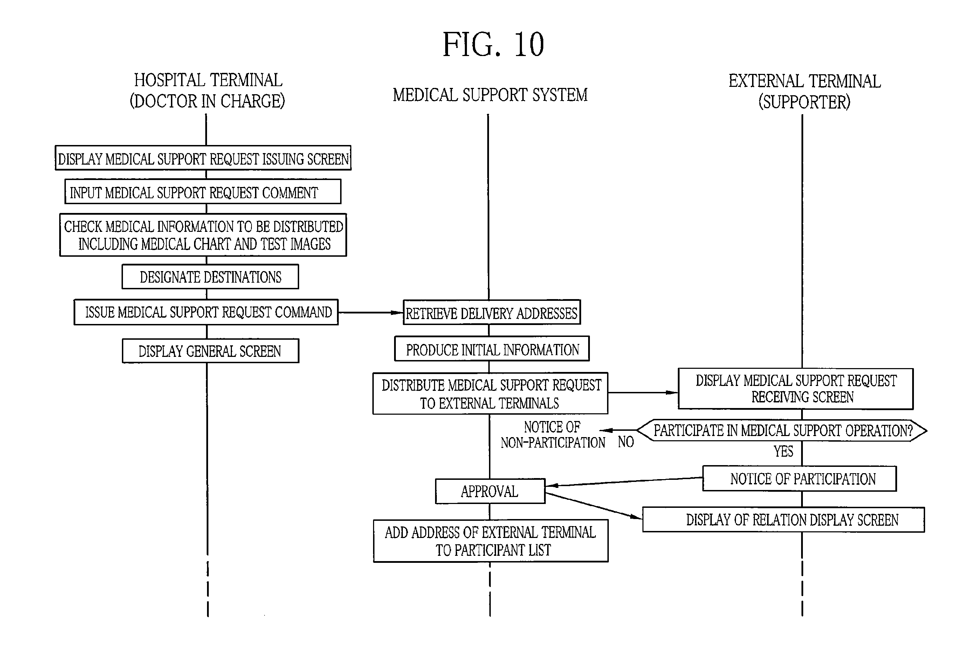

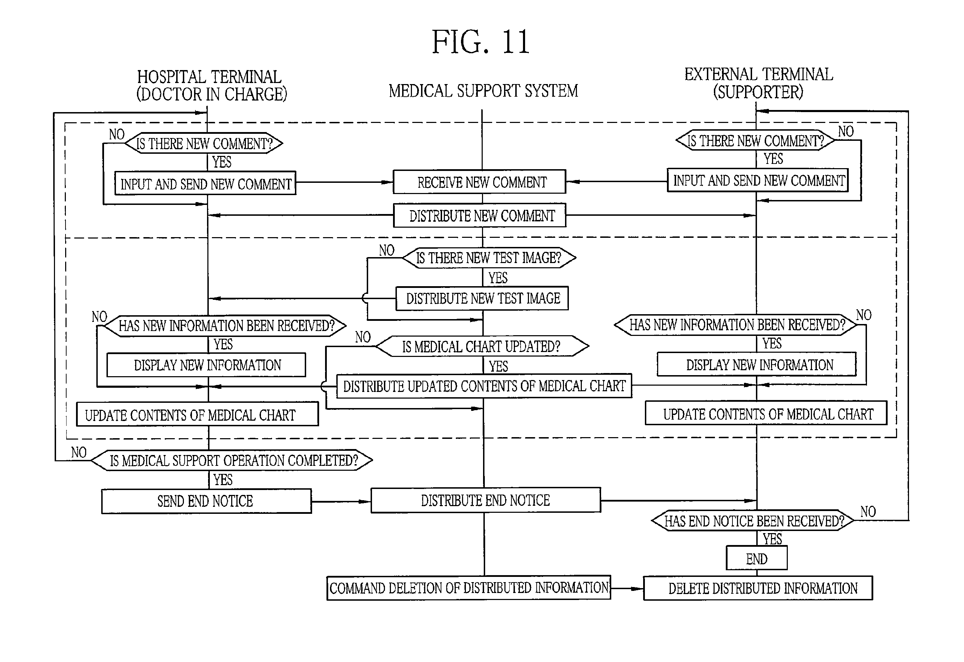

Next, the operation of the medical support system 10 will be described with referring to Figs. 10 to 13. The medical support operation by the medical support system 10 is used, for example, when the doctor desires to have advice from the medical specialists about the diagnosis and treatment of the patient. The patient may be an outpatient or an inpatient.

When the medical support operation is required, the doctor performs predetermined operation in the electronic medical chart screen on the hospital terminal 24 so as to display the medical support request issuing screen 51, as shown in Fig. 4. The patient ID and the reception ID are retrieved from the electronic medical chart, so it is unnecessary for the doctor to manually input or select the patient ID and the reception ID.

The doctor inputs the medical support request comment to the comment input box 52 of the medical support request issuing screen 51. The medical support request comment can have arbitrary contents, but includes a reason for needing the medical support operation and a matter in question, for example. Then, the doctor clicks the medical chart view button 53 to check the contents of the electronic medical chart to be distributed. The hospital terminal 24 retrieves the contents of the electronic medical chart to be distributed from the electronic medical chart server 22 with the use of the reception ID as a key, and displays the contents of the electronic medical chart on its monitor 24a. If the contents of the electronic medical chart are insufficient, the doctor inputs additional description to the electronic medical chart or to the comment input box 52 as the medical support request comment. The doctor sets at the importance setting field 52a the degree of importance to be attached to the medical support request comment inputted to the comment input box 52 on a scale of 1 to 10. Note that, the initial value of the degree of importance is set at "7". When the doctor does not set the degree of importance, the degree of importance to be attached to the comment is set at "7".

The test image list 54 includes the image IDs of the test images that have already been obtained. To check the test image, the doctor clicks the view button 54a. Upon a click of the view button 54a, the test image corresponding to the image ID is retrieved from the test image server 23 and displayed. Note that, when the doctor judges that the distribution of the test image is unnecessary, the delete button 54b is clicked to delete the image ID from the test image list 54.

To distribute a past test image that was obtained in the past, the add button 54c is clicked. Upon a click of the add button 54c, the test image selection screen pops up. One or more desired test images are selected in the test image selection screen, and the image IDs of the selected test images are added to the test image list 54. The doctor can change the degree of importance of each test image from its initial value "7" at the importance setting field 54d.

The doctor designates the destinations of the medical support request, e.g. the medical license or the specialization of the supporters to which the medical support request is to be sent, and then clicks the submit button 56. Thus, the medical support request, which includes the patient ID, the reception time, the reception ID, the elapsed time since the occurrence of the event, the medical support request comment, the test images and the image IDs attached thereto, is sent from the hospital terminal 24 to the medical support device 25. The name of the doctor who made the medical support request is sent too. After submitting the medical support request, the general screen 88 is displayed on the monitor 24a of the hospital terminal 24, as shown in Fig. 9.

When the medical support device 25 receives the medical support request, the destinations designated by the medical support request are sent to the management server 33. The management server 33 accesses its database 33a and retrieves the addresses of the supporters corresponding to the designated destinations based on a supporter registration list.

For example, when "surgeons and physicians" is designated as the destination, the addresses of the supporters who have a medical license of a surgeon or a physician are retrieved. When "gastroenterologist" is designated as the destination, the addresses of the supporters whose specialization is gastroenterology are retrieved. The retrieved addresses are sent to the information receiving section 39b of the distribution server 35. Note that, when "all supporters" is designated as the destination, the addresses of all the supporters registered in the database 33a are retrieved.

The reception ID, the elapsed time, the medical support request comment, and the image IDs, which are included in the medical support request, are sent to the information receiving section 39b of the distribution server 35. The information receiving section 39b retrieves the contents of the electronic medical chart from the electronic medical chart server 22 by using the reception ID as a key, and retrieves the test images corresponding to the image IDs from the test image server 23. After that, the contents of the electronic medical chart, the patient ID, the reception time, the reception ID, the elapsed time, the test images and their image IDs, the medical support request comment, and the doctor's name compose the initial information. Then, the information sending section 39a of the distribution server 35 distributes the medical support request including the initial information. The medical support request comment included in the initial information is attached with the importance information set at the importance setting field 52a.

The medical support request including the initial information is sent to the retrieved addresses, in other words, the external terminals 11 of the supporters designated in the medical support request issuing screen through the firewall 27 and the network 12. The contents of the medical support request including the initial information are stored as the distributed information.

Upon receiving the medical support request, the medical support request receiving screen 58 is displayed on the external terminals 11, as shown in Fig. 5.

The supporter checks the medical support request comment 61 and the degree of importance thereof and the contents of the electronic medical chart 62 displayed in the medical support request receiving screen 58 on the external terminal 11. The supporter further checks the test images as necessary, by clicking the view buttons 63a of the test image list 63. The degree of importance of each test image is checked at the importance showing field 63b.

The supporter determines participation or non-participation in the medical support operation in accordance with whether or not to take part in the medical support operation or whether or not to check progress, and clicks the participation button 65 or the non-participation button 66. Upon a click of the non-participation button 66, the external terminal 11 notifies the medical support device 25 of the non-participation. After that, the comments and the like made in the medical support operation are not distributed to the external terminal 11. After the notification of the non-participation, the medical support request receiving screen 58 is closed.

Upon a click of the participation button 65, on the other hand, the authorization screen (not shown) is displayed on the monitor 11a. The supporter enters the predetermined authorization ID and the password to the authorization screen using the keyboard or the like.

Accordingly, a notice of the participation is sent from the external terminal 11 to the medical support device 25 through the network 12 and the firewall 27, together with the entered authorization ID and the password.

The entered authorization ID and the password are received by the management server 33. The management server 33 identifies the external terminal 11 that has sent the authorization ID and the password based on the information received at this time. After that, it is judged that whether or not the authorization ID and the password entered from the external terminal 11 coincide with those corresponding to the identified external terminal 11.

When the authorization ID or the password does not coincide, the management server 33 notifies the supporter that the authorization ID or the password is wrong through the distribution server 35, and asks for re-entering the authorization ID and the password. Note that, if the database 33a does not have the information of the identified external terminal 11, the management server 33 notifies the supporter that the external terminal 11 is not registered.

When the authorization ID and the password coincide, the supporter is authorized to be the participant of the medical support operation. The address of the participant is sent to the information receiving section 39b of the distribution server 35, and is added to the participant list. Whenever receiving the authorization ID, the password, and the notice of the participation from the external terminal 11, the management server 33 performs above processing to identify the supporter, and adds the address of the authorized supporter to the participant list.

A normal completion notice of the identification is sent from the information sending section 39a to the external terminal 11 registered on the participant list. Upon receiving the normal completion notice, the relation display screen 68 is displayed on the monitor 11a of the external terminal 11, as shown in Fig. 6. Before the participants make any comment, only the comment box 74 displaying the medical support request comment 61 and the thumbnail images 76 of the test images registered in the test image list 54 are displayed in the information display section 73 of the relation display screen 68.

Upon receiving the normal completion notice, the red LED 11b provided in the external terminal 11 blinks. In addition to or instead of this, the speaker or the siren (neither shown) sounds or beeps. The blinks, sound, or beep inform the people around the supporter of the participation in the medical support operation.

To check the contents of the medical support request comment 61 in the relation display screen 68, the comment box 74 is clicked and the comment screen 81 is opened. To check the test image, the thumbnail image 76 is clicked and the test image screen 86 is opened. To check the contents of the electronic medical chart, the medical chart view button 78 is clicked and the contents of the electronic medical chart are displayed.

The supporters can evaluate whether or not the medical support request comment 61 displayed in the comment box 74 is important by clicking the evaluation buttons 74a and 74b provided in the relation display screen 68. Also, the supporters can evaluate whether or not the test image of the thumbnail image 76 is important by clicking the evaluation buttons 76a and 76b provided in the relation display screen 68.

Information about who has clicked the evaluation buttons 74a and 74b and how many times the evaluation buttons 74a and 74b have been clicked is sent from the external terminal 11 of each supporter to the importance determining section 37 of the distribution server 35. The importance information on the medical support request comment set by the doctor and the importance information on the medical support request comment set by each supporter using the evaluation buttons 74a and 74b are collected to the distribution server 35. An average degree of importance of the medical support request comment is calculated from the collected importance information. The average degree of importance is calculated as an average value of the degrees of importance set by the doctor and the supporters, for example. Whenever the evaluation button 74a or 74b is clicked, the degree of importance set by the supporter is sent and collected, and the average degree of importance is calculated. The calculated average degree of importance is sent to the terminals 11 and 24. The display control section 36 commands each terminal 11 or 24 to change the display style of the medical support request comment in accordance with variation in the average degree of importance. In response to this command, the display style of the medical support request comment is changed.

Information about who has clicked the evaluation buttons 76a and 76b and how many times the evaluation buttons 76a and 76b have been clicked is sent from the external terminal 11 of each supporter to the importance determining section 37 of the distribution server 35. The importance information on each test image set by the doctor and the importance information on each test image set by each supporter using the evaluation buttons 76a and 76b are collected to the distribution server 35. An average degree of importance on each test image is calculated from the collected importance information. The average degree of importance is calculated as an average value of the degrees of importance set by the doctor and the supporters, for example. Whenever the evaluation button 76a or 76b is clicked, the degree of importance set by the supporter is sent and collected, and the average degree of importance is calculated. The calculated average degree of importance is sent to the terminals 11 and 24. The display control section 36 commands each terminal 11 or 24 to change the display style of the test image in accordance with variation in the average degree of importance. In response to this command, the display style of each test image is changed.

To make a comment, the participant clicks the comment button 77. In response to the click of the comment button 77, the comment input screen is displayed. In the comment input screen, the participant inputs the comment e.g. his/her advice, findings, and the like to the comment input field 83 and sets the degree of importance of the comment at the importance setting field 83a, and then clicks the send button 84. Thus, the comment is sent to the medical support device 25 together with its importance information.

The comment and its importance information sent from the external terminal 11 are received by the information receiving section 39b of the distribution server 35. When the degree of importance of the comment equals or exceeds "7", being a threshold value, the reception state setting section 38 temporarily suspends the transmission of new medical information such as another comment from the external terminals 11 of the participants and the hospital terminal 24 of the doctor, which are to receive the distribution of the comment. After that, the information sending section 39a distributes the comment to the external terminals 11 of the participants and the hospital terminal 24 of the doctor. The distributed comment is stored in the database 35a as the distributed information. In the external terminals 11 and the hospital terminal 24, the information display section 73 is updated. To be more specific, a new comment box 75 displaying the comment is displayed in a manner related to the referred medical support request comment or test image. The relation display controller 36b manages the relation between the comment boxes 74 and 75, and between the comment box 75 and the test image 76.

The comment box 75 has the evaluation buttons 75a and 75b to evaluate the contents of the comment displayed in the comment box 75. The participant can evaluate the contents of the comment as the important or unimportant information by a click of the evaluation button 75a or 75b. The information about the evaluation is sent from each terminal 11 or 24 to the importance determining section 37 of the distribution server 35, and the average value of the degrees of importance is calculated. Whenever a new comment is issued from the external terminal 11 or the hospital terminal 24, the information display section 73 is updated, and a new comment box 75 having the evaluation buttons 75a and 75b is displayed. Also, the calculated average degree of importance of the medical information is sent to and displayed in each terminal 11 or 24. By a click of the new comment box 75, the comment screen 81 as shown in Fig. 7 is opened, and the distributed comment can be seen in detail.

Information about who has clicked the evaluation buttons 75a and 75b and how many times the evaluation buttons 75a and 75b have been clicked is sent from the external terminal 11 of each supporter to the importance determining section 37 of the distribution server 35. The importance information on the comment set by a commenter and the importance information on the comment set by each supporter using the evaluation buttons 75a and 75b are collected to the distribution server 35. An average degree of importance of the comment is calculated from the collected importance information. The average degree of importance is calculated as an average value of the degrees of importance set by the doctor and the supporters, for example. Whenever the evaluation button 75a or 75b is clicked, the degree of importance set by the supporter is sent and collected, and the average degree of importance is calculated. The calculated average degree of importance is sent to the terminals 11 and 24. The display control section 36 commands each terminal 11 or 24 to change the display style of the comment in accordance with variation in the average degree of importance. In response to this command, the display style of the comment is changed.

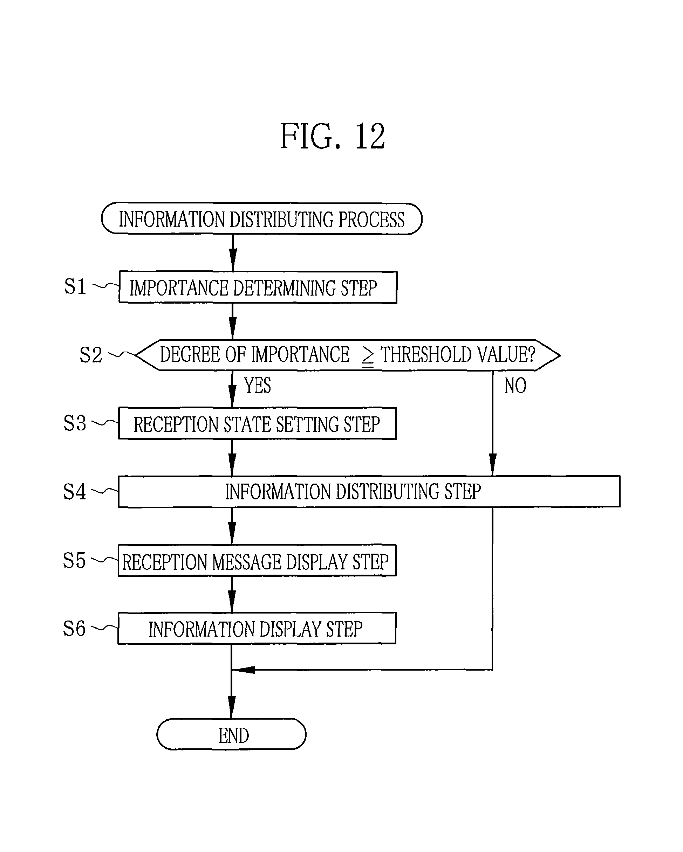

On the other hand, when the test image server 23 detects based on a patient ID attached to a test image obtained by the modality 31 that this test image belongs to the patient under the medical support operation, this test image is automatically attached with a degree of importance of "7", and is sent to the information receiving section 39b of the distribution server 35 together with the importance information. Since the degree of importance attached to this test image is "7" equaling to the threshold value (an importance determining step S1 and a judgment step S2 of Fig. 12), the reception state setting section 38 temporarily suspends the transmission of the medical image such as a comment from the external terminals 11 of the participants and the hospital terminal 24 of the doctor that are to receive the test image (a reception state setting step S3 of Fig. 12). The information sending section 39a distributes the test image to the external terminals 11 of the participants and the hospital terminal 24 of the doctor (an information distribution step S4 of Fig. 12). After the distribution of the test image to each terminal 11 or 24, the display control section 36 commands each terminal to display a message indicating the completion of reception of the test image (a reception message display step S5 of Fig. 12). In response to this command, the completion of the reception of the test image is displayed in the terminals 11 and 24. The display control section 36 commands each terminal 11 or 24 to automatically display the test image (an information display step S6 of Fig. 12). In response to this command, the test image is displayed on the monitor 11a or 24a of each terminal 11 or 24. Also, in the information display section 73 displayed on the monitor 11a or 24a of each terminal 11 or 24, a thumbnail image 76 of the distributed test image is displayed together with the evaluation buttons 76a and 76b. A click of the thumbnail image 76 opens the test image screen 86, as shown in Fig. 8, to show the distributed test image.

The participants can evaluate the test image as the important or unimportant information by a click of the evaluation button 76a or 76b. Information about the evaluation is sent to the importance determining section 37 of the distribution server 35, and processing similar to that applied to the test image distributed at the time of issuing the medical support request is performed. Whenever a new test image of the patient is obtained by the modality 31, the new test image is distributed in a similar procedure, and a thumbnail image 76 of the new test image is displayed in the information display section 73.

A new comment can be made in the comment screen 81 or the test image screen 86, as with above. A commenter can set the degree of importance to this comment, as with above.

When the doctor updates the contents of the electronic medical chart from the hospital terminal 24, the electronic medical chart server 22 updates the medical chart information on the database 22a. The new contents of the electronic medical chart are sent to the information receiving section 39b of the distribution server 35. The information sending section 39a of the distribution server 35 distributes the new contents of the electronic medical chart to the external terminals 11 of the participants. Therefore, the participants check the updated contents of the electronic medical chart.

An information distribution process performed by the distribution server 35 will be described with referring to a flowchart of Fig. 12. In the above description, "information" denotes the medical information including the comments from the participants, image data obtained by the modality 31 and managed by the test image server 23, the comments from the doctor, and the medical chart information updated by the doctor.

The importance determining section 37 performs the importance determining step S1 for determining the degree of importance of the information, and the judgment step S2 for judging whether or not the degree of importance equals or exceeds the predetermined threshold value. When the degree of importance of the information is the threshold value or more, the reception state setting section 38 temporarily suspends the transmission of other information from the external terminals 11 and the hospital terminal 24 that are to receive the important information, and sets the external terminals 11 and the hospital terminal 24 in the ready-to-receive state (the reception state setting step S3). After that, the information sending section 39a distributes the important information (information distribution step S4). After the distribution of the information to each terminal 11 or 24, the display control section 36 commands each terminal 11 or 24 to display the message indicating the completion of reception of the test image (the reception message display step S5). In response to this command, the completion of the reception of the test image is displayed in the terminals 11 and 24. The display control section 36 commands each terminal 11 or 24 to automatically display the information (the information display step S6). In response to this command, the information is displayed on the monitor 11a or 24a of each terminal 11 or 24. On the other hand, when the degree of importance is less than the threshold value in the judgment step S2, the information is distributed to the terminals 11 and 24 without being put in the ready-to-receive state in the information distribution step S4.

In the importance determining step S1, the degree of importance of the medical information set by the doctor or commenter himself/herself, the degree of importance set based on initial setting, and the degree of importance of the medical information set by each participant are sent to the importance determining section 37 of the distribution server 35. In other words, all types of importance information are collected to the distribution server 35. An average value of the collected degrees of importance is calculated, and the average value is determined as the general degree of importance of the medical information. At this time, the average value may be calculated from the collected degrees of importance that are weighted in accordance with a title or experience of the commenter or the participants.

In the judgment step S2, the degree of importance (the average degree of importance) calculated in the importance determining step S1 is compared with the threshold value. In the reception state setting step S3, all the external terminals 11 and the hospital terminal 24 that are to receive the important information are put in the ready-to-receive state. In the information distribution step S4, the important information is distributed to the external terminals 11 and the hospital terminal 24 that are in the ready-to-receive state.

In the distribution of the medical support request having the medical information including the medical support request comment and the test images inputted by the doctor from the hospital terminal 24, the reception state setting step S3 is not carried out. Also in this case, the display style is preferably determined in an appropriate manner in accordance with a judgment result of the judgment step S2.

Since the distribution server 35 has the function of the information distribution process shown in Fig. 12, it is possible to quickly and reliably distribute the important information to all the external terminals 11 and the hospital terminal 24 that should receive the importance information. The display style is determined in accordance with the degree of importance of the medical information, so it is possible to recognize the degree of importance of the medical information at sight, and prevent missing the important information. Therefore, the medical information can be shared among the doctor and the participants quickly and reliably, and facilitate performing the medical support operation.

Out of the medical information included in the medical support request, the medical information that should be shared among the doctor and the participants is distributed as the important information. Thereby, it is possible to teach medical knowledge from the doctor to the participants. When some participants have expert knowledge, distributing comments from the expert participants with a high degree of importance makes it possible to perform medical education to the doctor and the other participants.

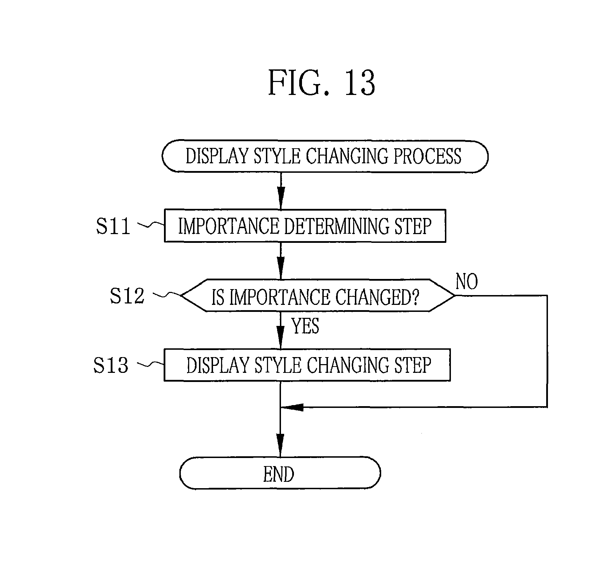

Furthermore, the distribution server 35 has the function of changing the display style of the medical information, when the degree of importance of the distributed information is increased or decreased by one level or more by clicks of the evaluation buttons. A display style change process will be described with referring a flowchart of Fig. 13. The display style change process includes an importance determining step S11, a variation judging step S12, and a display style changing step S13. In the importance determining step S11, the average degree of importance of the medical information after being changed by clicks of the evaluation buttons by the participant is detected. In the variation judging step S12, the difference between the average degree of importance of the medical information detected in the step S11 and the initial degree of importance of the medical information set by the sender is calculated. The calculated difference is determined as variation in the importance information. In the display style changing step S13, when the variation in the importance information determined in the step S12 is 1 or more, the change of the display style is commanded to each terminal 11 or 24. Each terminal 11 or 24 changes the display style in response to this command.

In the importance determining step S11, the importance determining section 37 re-determines the degree of importance of the medical information in accordance with the number of clicks of the evaluation buttons 74a, 74b, 75a, 75b, 76a, 76b, 82a, 82b, 87a, and 87b of the participants, in addition to the initial degree of importance set at the time of distribution of the medical information.

In the variation judging step S12, the importance determining section 37 compares the degree of importance determined in the importance determining step S11 with the initial degree of importance set at the time of the distribution. When the variation in the degree of importance is one level or more, the display style changer 36a performs the display style changing step S13.

In the display style changing step S13, a display style, which designates the presence or absence of blinking, color, a typeface, and a font size, is appropriately changed. For example, when the medical information is text, one of the following four display styles is adopted.

According to a first display style, if the degree of importance is "0" or more and less than "7", the text just lights up. If the degree of importance is "7" or more and "10" or less, the text blinks. When the degree of importance changes from "5" to "7", for example, the text changes from a lighting state into a blinking state.

According to a second display style, color of the text is changed in accordance with the degree of importance. For example, if the degree of importance is "0" or more and less than "3", the text color is blue. If the degree of importance is "3" or more and less than "6", the text color is green. If the degree of importance is "6" or more and less than "8", the text color is yellow. If the degree of importance is "8" or more and less than "9", the text color is orange. If the degree of importance is "9" or more and "10" or less, the text color is red. When the degree of importance changes from "5" to "7", the text color is changed from green to yellow.

According to a third display style, a typeface of the text is changed in accordance with the degree of importance. For example, if the degree of importance is "0" or more and less than "5", a normal typeface is used. If the degree of importance is "5" or more and less than "7", an italic typeface is used. If the degree of importance is "7" ore more and "10" or less, a bold typeface is used. When the degree of importance changes from "5" to "7", the italic typeface is changed to the bold typeface.

According to a fourth display style, a font size is changed in accordance with the degree of importance. For example, if the degree of importance is "0" or more and less than "4", the font size is set at 8 pt. If the degree of importance is "4" or more and less than "7", the font size is set at 10 pt. If the degree of importance is "7" or more and less than "9", the font size is set at 12 pt. If the degree of importance is "9" ore more and "10" or less, the font size is set at 16 pt. When the degree of importance is changed from "5" to "7", the font size is changed from 10 pt to 12 pt.

In a case where the medical information is the test image, a frame enclosing the thumbnail image of the test image blinks or just lights up, and the color of the frame is changed. Alternatively, the display of text information attached to the test image may be changed, as in the case of the text described above.

Since the distribution server 35 has the function of changing the display style, it is possible to prevent missing the especially important information. Note that, after the display style has been changed, the variation judging step S12 is performed using the degree of importance at the time of immediately preceding change of the display style as comparison reference, instead of using the degree of importance at the time of the distribution of the medical information.

The elapsed time controller 36c can detect elapsed time since the terminal 11 or 24 received unviewed medical information on a terminal-by-terminal basis. The degree of importance of the unviewed medical information may be varied in accordance with the elapsed time on a terminal-by-terminal basis. Thus, the more time elapses, the more important information the display style of the unviewed medical information changes to that for. This prevents the participant from missing the medical information more reliably. Whether the medical information is viewed or unviewed is detected by the viewed number detecting section 40.

The viewed number detecting section 40 detects unviewed medical information on a terminal-by-terminal basis, and how many times the unviewed medical information has been viewed in the other terminals. In accordance with the viewed number, the degree of importance of the unviewed medical image may be varied on a terminal-by-terminal basis. Thereby, the display style of the unviewed medical information is changed to that suited for more important information with increase in the number of viewed by the other participants. This prevents the participant from missing the medical information more reliably.

When the participant has not viewed the important information yet, a warning may be displayed in an area provided in a corner of the information display section 73, the comment screen 81, and the test image screen 86. A display style of this warning, including the presence or absence of blinking, color, a typeface, and a font size, is changeable in accordance with the degree of importance of the unviewed medical information, as with above.

Note that, it is preferable that each participant can customize a display format of the distributed medical information to some extent. For example, the position, the size, and the like of the information display section 73, the comment screen 81 and the test image screen 86 can be customized to improve the convenience of the participant.

The medical support operation is carried out as described above. The doctor and the participants exchange opinions with one another with referring to the comments and the test images displayed in the information display section 73. Also, the participants can give advice to the doctor. Thus, the doctor can make proper diagnosis and treatment.

According to the medical support device and system of the present invention, the distributed information is displayed in the external terminals 11 and the hospital terminal 24 in a related manner. The updated medical information is automatically updated and distributed, so it is possible to actualize favorable medical support environment. Furthermore, since the distribution method and the display style of the medical information are changed in accordance with the degree of importance of the medical information, the important information can be reliably distributed and viewed by the doctor and the participants without being missed. Using this medical support device and system allows performing appropriate medical practice at appropriate time.

When the participant leaves the ongoing medical support operation, the participant clicks the end button 79. Thus, the leave notice is sent from the external terminal 11 of the participant to the medical support device 25, and the information sending section 39a of the distribution server 35 deletes the address of the participant from the participant list. Therefore, the medical information is not distributed to the external terminal 11 that has sent the leave notice. The terminal control server 34 deletes through the network 12 the medical information that was distributed and stored in the leaving external terminal 11.

When the treatment has been completed owing to the adequate medical support operation, the doctor operates the end button 93 in the hospital terminal 24 to end the medical support operation. In response to this operation, the end notice is sent to the medical support device 25. The end notice is sent from the information sending section 39a of the distribution server 35 to the external terminals 11 of the participants, and the terminal control server 34 deletes through the network 12 the medical information that was distributed and stored in the external terminals 11.

The present invention is effectively applicable in an emergency medical situation, for example. The external terminal 11 may be the portable telephone, the smartphone, or the like. In such a case, it is preferable to change the display format and the like of the medical support request receiving screen 58, the relation display screen 68, the information display section 73, the comment input screen, the comment screen 81, and the test image screen 86 in an appropriate manner.

In the above embodiment, a certain medical license e.g. "surgeons and physicians" is designated, the management server 33 retrieves the supporters having the designated medical license from a list of all the supporters. Instead of this, before issuing the medical support request, the management server 33 may send a list of the supporters having the designated medical license to the hospital terminal 24 of the doctor. The doctor chooses one or more suitable supporters from the list of the supporters having the designated medical license. The management server 33 retrieves the addresses (terminal addresses) of the chosen supporters.

Although the present invention has been fully described by the way of the preferred embodiment thereof with reference to the accompanying drawings, various changes and modifications will be apparent to those having skill in this field. Therefore, unless otherwise these changes and modifications depart from the scope of the present invention, they should be construed as included therein.

## Claims

1. A medical support device (25) connected to a hospital terminal (24) operated by a doctor in charge of treatment, an electronic medical chart server (22) for providing medical chart information, and a test image server (23) for providing a test image, and connected through a network (12) to an external terminal (11) that a supporter has, said medical support device (25) comprising:
a distribution server (35) for distributing a participation request in a medical support operation to said supporter in response to a medical support request command sent by said doctor from said hospital terminal (24), and for distributing medical information including said medical chart information and said test image to said doctor and said supporter, said distribution server (35) including:
an importance determining section (37) for determining a degree of importance of said medical information, said degree of importance being set at a predetermined initial value and changeable using said hospital terminal (24) or said external terminal (11); and
a reception state setting section (38) for temporarily putting said hospital terminal (24) and said external terminal (11) in a transmission disabled state and enabling only reception of said medical information, when said degree of importance of said medical information to be sent equals or exceeds a threshold value.

2. The medical support device (25) according to claim 1, further comprising:
a management server (33) for registering and deregistering said supporter, and approving participation of said supporter in said medical support operation; and
a terminal control server (34) for controlling operation of said hospital terminal (24) and said external terminal (11) through a medical support application installed in said hospital terminal (24) and said external terminal (11).

3. The medical support device (25) according to claim 1, wherein said distribution server (35) has a reception display command section (36) for commanding said hospital terminal (24) or said external terminal (11) to automatically display a message indicating completion of reception of said medical information on a monitor (24a, 11a) of said hospital terminal (24) or said external terminal (11).

4. The medical support device (25) according to claim 1, wherein said distribution server (35) has an information display command section (36) for commanding said hospital terminal (24) or said external terminal (11) to automatically display said medical information received from said distribution server (35) on a monitor (24a, 11a) of said hospital terminal (24) or said external terminal (11).

5. The medical support device (25) according to claim 3, wherein said reception display command section (36) commands at least one of: to blink said message indicating completion of reception, to change color of said message indicating completion of reception, to change a typeface of said message indicating completion of reception, and to change a font size of said message indicating completion of reception.

6. The medical support device (25) according to claim 4, wherein said information display command section (36) commands at least one of: to blink display of said received medical information, to change color of said display of said received medical information, to change a typeface of said display of said received medical information, and to change a font size of said display of said received medical information.

7. The medical support device (25) according to one of claims 1 to 6, wherein a display format of said hospital terminal (24) or said external terminal (11) is customized by a user of said hospital terminal (24) or said external terminal (11).

8. The medical support device (25) according to one of claims 1 to 7, wherein condition description or medical education is carried out between said doctor and said supporter using said medical information having said degree of importance that equals or exceeds said threshold value.

9. A medical support system (10) comprising:
(A) a hospital terminal (24) operated by a doctor in charge of treatment;
(B) an electronic medical chart server (22) for providing medical chart information;
(C) a test image server (23) for providing a test image;
(D) an external terminal (11) that a supporter has; and
(E) a medical support device (25) connected to said hospital terminal (24), said electronic medical chart server (22), and said test image server (23), and connected through a network (12) to said external terminal (11), said medical support device (25) including:
a distribution server (35) for distributing a participation request in a medical support operation to said supporter in response to a medical support request command sent by said doctor from said hospital terminal (24), and for distributing medical information including said medical chart information and said test image to said doctor and said supporter, said distribution server (35) including:
an importance determining section (37) for determining a degree of importance of said medical information, said degree of importance being set at a predetermined initial value and changeable using said hospital terminal (24) or said external terminal (11); and
a reception state setting section (38) for temporarily putting said hospital terminal (24) and said external terminal (11) in a transmission disabled state and enabling only reception of said medical information, when said degree of importance of said medical information to be sent equals or exceeds a threshold value.
